# NOUVEAU FASCICULE DE BREVET EUROPEEN

(11) **EP 2 988 756 B2**
(45) Date de publication et mention de la décision concernant l'opposition: **18.05.2022**
(45) Mention de la délivrance du brevet: 08.03.2017
(21) Numéro de dépôt: 14723750.7
(22) Date de dépôt: 28.04.2014
(51) Int. Cl.: A61K 33/24, A61K 33/06, A61K 49/06, A61K 49/10, A61K 9/08, A61K 51/04

(54) **FORMULATION DE PRODUIT DE CONTRASTE ET SON PROCEDE DE PREPARATION ASSOCIE**
KONTRASTMITTELFORMULIERUNG UND ZUGEHÖRIGES HERSTELLUNGSVERFAHREN
CONTRAST MEDIUM FORMULATION AND RELATED PREPARATION METHOD

(30) Priorité: 26.04.2013 FR 1353883
(43) Date de publication de la demande: 02.03.2016
(73) Titulaire: Guerbet, 93420 Villepinte (FR)
(72) Inventeur: MEDINA, Christelle, F-77360 Vaires Sur Marne (FR); SABATOU, Monique, F-93110 Rosny-sous-Bois (FR); PETIT, Anne, F-28300 Leves (FR); PORT, Marc, F-95170 Deuil La Barre (FR)
(74) Mandataire: Regimbeau
(86) Numéro de dépôt international: PCT/EP2014/058617
(87) Numéro de publication internationale: WO 2014/174120

(56) Documents cités:
- EP-A1- 2 554 167
- EP-A2- 0 454 078
- EP-B1- 1 931 673
- WO-A1-89/00052
- WO-A2-2009/103744
- FR-A1- 2 891 830
- FR-A1- 2 927 539
- US-A- 5 876 695

## Description

L'invention concerne des formulations d'agents de contraste, en particulier de chélates d'ions de métaux paramagnétiques, notamment pour l'Imagerie par Résonance Magnétique, et des procédés d'obtention performants sur le plan industriel de ces formulations.

On connaît de nombreux agents de contraste à base de chélates de lanthanides (métal paramagnétique), en particulier de gadolinium, décrits par exemple dans le document US 4 647 447. Ces produits sont souvent rassemblés sous le terme GBCA (Gadolinium-based Contrast Agent, produits de contraste à base de gadolinium). Plusieurs produits sont commercialisés, notamment à base de chélates macrocycliques, tels que le gadotérate DOTA (acide 1,4,7,10-tétraazacyclododécane-N,N',N",N"'-tétraacétique), le gadotéridol HPDO3A et le gadobutrol DO3A-butrol, et des chélates linéaires tels que le DTPA (acide diéthylènetriaminepentaacétique), le DTPA-BMA (gadodiamide) ou le BOPTA (gadobémate). Ces composés seront qualifiés indifféremment dans la suite du texte de « chélates » ou de « ligands chélateurs ».

D'autres produits, dont certains sont en cours de développement, représentent une nouvelle génération de GBCA. On peut notamment citer les complexes de chélates macrocycliques comme l'acide bicyclopolyazamacrocyclocarboxylique (EP 0 438 206) ou de chélates macrocycliques dérivés de PCTA (c'est-à-dire comprenant a *minima* la structure chimique de l'acide 3,6,9,15-tétraazabicyclo[9,3,1]pentadéca-1(15),11,13-triène-3,6,9-triacétique), comme décrits dans les documents WO 93/11800, US 5 403 572, US 6 440 956 ou EP 1 931 673.

Les complexes de ligands chélateurs dérivés de PCTA décrits dans le document EP 1 931 673 ont notamment comme avantage d'être relativement faciles à synthétiser chimiquement et de présenter une relaxivité supérieure aux autres GBCA (relaxivité r₁ pouvant aller jusqu'à 11-12 mM⁻¹.s⁻¹ dans l'eau) actuellement sur le marché, cette relaxivité correspondant à l'efficacité de ces produits donc à leur pouvoir contrastant.

Les chélates de lanthanide sont en situation d'équilibre chimique. Il existe donc un risque de libération non souhaitée de métal paramagnétique. L'homme du métier est ainsi amené à rechercher des solutions techniques limitant ce risque pour résoudre de manière complétement sécurisée le problème technique complexe de la tolérance chez le patient, notamment lorsque le métal paramagnétique est le gadolinium. Ce problème est d'autant plus délicat que l'administration d'agents de contraste est souvent répétée lors d'examens de diagnostic et/ou pour le guidage et le suivi de l'efficacité d'un traitement thérapeutique.

Le problème complexe de la tolérance des nouveaux GBCA doit toujours être considéré, notamment dans des situations à risque de tolérance plus prononcé pour l'administration de produits de contraste IRM. Depuis 2006, une pathologie appelée NSF (Nephrogenic Systemic Fibrosis, fibrose néphrogénique systémique ou dermopathie fibrogénique), a été liée, au moins en partie, à l'existence de gadolinium dans l'organisme. Cette maladie a conduit à une alerte d'autorités de santé vis-à-vis d'agents de contraste gadolinés commercialisés pour certaines catégories de patients.

En définitive, ce problème technique de la tolérance de chélates de lanthanides reste complexe et important.

Une première stratégie de limitation de ce risque est de sélectionner des complexes qui possèdent des stabilités thermodynamique et cinétique les plus élevées possible. En effet, plus les stabilités du complexe sont élevées, plus la quantité de lanthanide libérée au cours du temps sera limitée.

Plusieurs autres axes d'amélioration de la tolérance de chélates de gadolinium sont décrits dans l'art antérieur. Le document US 5 876 695 divulgue des formulations comprenant un excès de chélate libre, destiné à compenser une libération non souhaitée du lanthanide, le chélate en excès venant complexer le lanthanide (par exemple le gadolinium) libéré. Le document US 5 876 695 décrit en particulier un excès de chélate linéaire, en particulier de DTPA libre. Cette stratégie de formulation est utilisée pour des produits tels que Magnevist^{®}, Vasovist^{®} ou Primovist^{®}. Le document WO 2009/103744 décrit une stratégie de formulation similaire, reposant sur l'ajout d'une quantité précise de chélate libre, de façon à avoir un excès très faible dudit chélate et une concentration nulle de lanthanide libre.

Les documents EP 0 454 078, US 5 876 695 et US 2004/0170566 décrivent l'utilisation de complexes « faibles » d'un ligand macrocyclique ou linéaire avec un métal ou un alcalino-terreux, notamment le calcium, sodium, zinc, magnésium. Ces complexes « faibles » subissent une transmétallation en présence de lanthanide libre, puisque les complexes entre lesdits ligands macrocycliques ou linéaires et un lanthanide, notamment le gadolinium, sont plus « forts », c'est-à-dire qu'ils sont plus stables thermodynamiquement. Un échange a donc lieu entre le calcium, sodium, zinc ou magnésium et le lanthanide : ce dernier est piégé par le ligand sous forme de complexe, tandis qu'est libéré dans la solution le calcium, sodium, zinc ou magnésium. Cette stratégie de formulation est, elle, utilisée pour des produits comme Gadovist^{®}, Omniscan^{®} ou OptiMark^{®}. Il est à noter que, dans les exemples de ces documents, le ligand du complexe dit « faible » est identique au ligand du complexe actif de gadolinium (i.e. utilisé comme produit de contraste). Ces documents mentionnent la possibilité d'utiliser deux ligands différents, à condition toutefois que la constante de stabilité du complexe « faible » soit plus basse que celle du complexe actif de gadolinium (voir notamment US 5,876,695 colonne 4 lignes 52-58).

Le demandeur a mené de nombreux travaux sur le cas spécifique des chélates macrocycliques et notamment des chélates dérivés de PCTA comme décrits dans EP 1 931 673. Il a tenté d'appliquer les différentes solutions connues en terme de formulation mais celles-ci se sont avérées impossibles à mettre en oeuvre ou économiquement non rentable dans le cas précis des complexes entre ces chélates dérivés de PCTA et des ions de métaux paramagnétiques, ou insatisfaisantes pour assurer que des ions de métaux paramagnétiques comme le gadolinium ne soient pas libérés tant au cours du procédé de fabrication du produit de contraste qu'au cours du stockage de ce produit, avant son utilisation chez un patient. Un élément important à prendre en compte est que ces complexes, tout en ayant une stabilité cinétique très importante, possèdent une constante thermodynamique faible.

Le Demandeur n'a déjà pas souhaité utiliser de ligands chélateurs linéaires puisque ces chélates ne sont pas suffisamment stables pour assurer une absence de libération d'ions de métaux paramagnétiques au cours de la vie du GBCA. Ensuite, le Demandeur a été surpris de constater que l'utilisation de chélates macrocycliques ayant des structures chimiques proches de celle du chélate ne permettait pas non plus d'assurer cette absence d'ions de métaux paramagnétiques dans la formulation. En se basant sur les formulations déjà existantes sur le marché ou sur l'enseignement de documents de l'art antérieur, un homme du métier serait fortement incité à n'utiliser pour la formulation de complexes de chélates de métal paramagnétique, que le ligand de ces complexes libre ou complexé avec du calcium.

Pour un homme du métier, l'utilisation d'un ligand chélateur libre ou sous forme de complexe de métal ou d'un alcalino-terreux comme le calcium qui présenterait une stabilité thermodynamique supérieure à celle du complexe à formuler n'est pas souhaitable puisqu'il y aurait le risque d'un échange de métal paramagnétique en faveur du chélate présentant une constante thermodynamique plus forte pour le métal paramagnétique.

C'est en surmontant ce préjugé technique que le Demandeur a pu apporter une solution au problème technique de la tolérance de chélates de métal paramagnétique dérivés de PCTA.

Le Demandeur a pu démontrer que des chélates macrocycliques, et plus spécialement le DOTA, ont un comportement différent de chélates linéaires tels que le DTPA en matière de tolérance résultant de l'excès de ligand. Toutefois, pour les raisons expliquées ci-dessus, un homme du métier aurait été dissuadé d'utiliser du DOTA pour la préparation d'une composition comprenant un complexe dérivé de PCTA.

En effet, le demandeur a découvert que l'ajout de sels de DOTA (acide 1,4,7,10-tetraazacyclododecane-1,4,7,10-tetraacetique) à une composition comprenant un complexe de métal paramagnétique d'un dérivé de PCTA permet d'assurer l'absence de libération de métal paramagnétique dans la formulation, notamment dans la solution injectable, aussi bien que dans le corps du patient après injection, tout en conservant les performances en tant que produit de contraste en imagerie médicale.

Un objet de la présente invention concerne donc une composition pharmaceutique liquide comprenant un complexe dérivé de PCTA et comprenant en outre un complexe calcique d'acide 1,4,7,10-tetraazacyclododecane-1,4,7,10-tetraacetique, préférentiellement un complexe d'acide 1,4,7,10-tetraazacyclododecane-1,4,7,10-tetraacetique mono-calcique (DOTA-Ca) ou un complexe d'acide 1,4,7,10-tetraazacyclododecane-1,4,7,10-tetraacetique dicalcique (DOTA-Ca₂), et ayant une concentration en métal paramagnétique libre inférieure à 1 ppm (m/v), préférentiellement inférieure à 0,5 ppm (m/v).

Dans la suite du texte, une façon alternative de désigner un complexe entre un ligand chélateur de formule (I) et un métal paramagnétique sera de le nommer « PCTA - métal paramagnétique ». Par exemple, sauf indication contraire, le complexe entre ce ligand chélateur de formule (I) et un ion de gadolinium sera désigné par « PCTA-Gd ». Un complexe entre du DOTA et du gadolinium sera désigné par « DOTA-Gd ».

Un autre objet de l'invention a trait à un produit de contraste pour l'imagerie médicale comprenant ladite composition.

Un autre objet de la présente invention concerne un procédé de préparation de ladite composition.

La présente invention concerne également ladite composition ou ledit produit de contraste pour leur utilisation dans une méthode de diagnostic.

Ainsi, l'invention se rapporte à une composition pharmaceutique liquide comprenant un complexe de formule (I) : 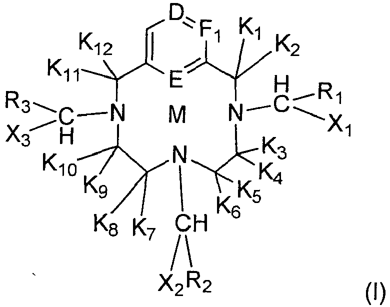
dans laquelle R₁, R₂ et R₃ représentent -COOH,
X₁, X₂ et X₃ représentent indépendamment l'un de l'autre L-Y dans laquelle L représente un groupe (CH₂)ₙ avec n = 1 à 3, Y représente -CO-NR₇R₈ dans laquelle R₇ représente H et R₈ représente un groupe -CH₂-CH₂OH, -CHOH-CH₂OH, -CH-(CH₂OH)₂, - (CH₂)-(CHOH)ₚ-CH₂OH avec p = 1 à 4 ou -C-(CH₂OH)₃ ;
D représente CH ;
E représente N ;
F₁ représente CH ;
K₁ à K₁₂ représentent chacun ; et
M représente Gd³⁺ ;
ou un énantiomère, ou un diastéréoisomère (préférentiellement choisi parmi les diastéréoisomères RRS, RSR, RSS) de ceux-ci ou leurs mélanges,
ladite composition comprenant en outre un complexe calcique d'acide 1,4,7,10-tetraazacyclododecane-1,4,7,10-tetraacetique, préférentiellement un complexe d'acide 1,4,7,10-tetraazacyclododecane-1,4,7,10-tetraacetique mono-calcique (DOTA-Ca) ou un complexe d'acide 1,4,7,10-tetraazacyclododecane-1,4,7,10-tetraacetique dicalcique (DOTA-Ca₂), et ayant une concentration en métal paramagnétique libre inférieure à 1 ppm (m/v), préférentiellement inférieure à 0,5 ppm (m/v).

De manière préférentielle, le complexe calcique d'acide 1,4,7,10-tetraazacyclododecane-1,4,7,10-tetraacetique selon l'invention sera un complexe d'acide 1,4,7,10-tetraazacyclododecane-1,4,7,10-tetraacetique mono-calcique.

Dans la suite, on notera DOTA-calcium ou DOTA-Ca le complexe calcique d'acide 1,4,7,10-tetraazacyclododecane-1,4,7,10-tetraacetique que ce soit le complexe monocalcique ou le complexe dicalcique.

La composition selon l'invention a comme avantage de présenter un risque inexistant, avant la date de péremption, de libération non souhaitée de métal paramagnétique et cela grâce au choix de formulation spécifique du complexe de formule (I) tel que défini précédemment en mélange avec le complexe DOTA-Calcium.

La composition selon l'invention présente ainsi une stabilité sur la durée, c'est-à-dire que sa composition reste conforme aux spécifications en termes de concentration de métal paramagnétique libre (en particulier sa concentration en métal paramagnétique libre reste inférieure à 1 ppm (m/v)), sur une durée d'au moins 3 ans, préférentiellement d'au moins 4 ans ou plus préférentiellement d'au moins 5 ans, notamment en termes de teneur en métal paramagnétique libre. Selon les directives ICH, une observation de cette stabilité pendant 6 mois à 40°C est considérée comme une bonne indication d'une stabilité de 3 ans à 25°C.

### Complexe de formule (I)

On préfère particulièrement les complexes de formule (I) pour lesquels les trois chaînes Y ont chacune un poids moléculaire inférieur à 200, avantageusement entre 50 et 100, et notamment les composés pour lesquels les chaînes Y comprennent chacune 1 à 5 groupes OH.

Selon l'invention, le complexe de formule (I) est tel que E représente un atome N et D et F₁ représentent CH.

Selon l'invention, le complexe de formule (I) est tel que X₁ à X₃ représentent indépendamment -(CH₂)n-CO-NR₇R₈ dans laquelle n est compris entre 1 et 3, R₇ représente H, R₈ représente -CH₂- CH₂OH, -CHOH-CH₂OH, -CH-(CH₂OH)₂, -CH₂-(CHOH)p-CH₂OH avec p =1 à 4 ou -C-(CH₂OH)₃.

Préférentiellement, le complexe de formule (I) est complexe entre un ligand formule (I') 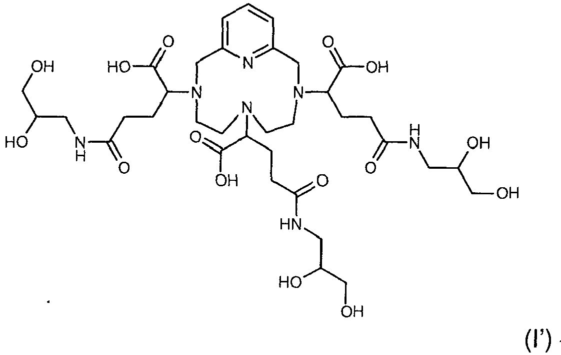 et un ion Gd³⁺.

Selon l'invention, l'ion de métal paramagnétique M est Gd³⁺.

Les complexes de formule (I) tels que définis précédemment ont une relaxivité (efficacité en imagerie) et une efficacité massique (prix de revient industriel) très nettement améliorées, avec notamment des valeurs de relaxivité r1 de l'ordre de 9 à 15 mM.s⁻¹.Gd⁻¹, c'est-à-dire des valeurs de relaxivité multipliées d'un facteur 2 à 3 par rapport à celles des dérivés notamment de DO3A, DOTA ou DTPA antérieurs. Ces composés sont bien adaptés à l'imagerie à haut champ magnétique (par exemple pour des champs de 3 Tesla). Les complexes de formule (I) présentent plusieurs caractéristiques fonctionnelles particulièrement performantes une fois combinées :
1. non ionicité : cela permet de limiter fortement l'osmolalité du produit à injecter et donc la dose de produit injectée, ce qui est une caractéristique importante pour les produits de contraste afin d'améliorer le confort des patients et de réduire le coût de l'injection ;
2. forte hydrophilie : cela permet une non toxicité et une solubilité appropriée du produit ;
3. haute relaxivité (haute intensité du signal) : la relaxivité est élevée et n'est pas altérée (non quenchée) par les groupes hydroxyles de la structure ;
4. faible prix de revient industriel (forte efficacité massique) ; et
5. faible masse moléculaire permettant d'obtenir une biodistribution de composé non spécifique : on évite un comportement par exemple non recherché de type Blood Pool Agent qui correspond à une diffusion sélective dans le compartiment vasculaire notamment.

Les procédés de synthèse de ces complexes de formule (I) sont bien connus de l'homme du métier, et sont notamment décrits dans le document EP 1 931 673.

Dans le cas particulier où M représente un ion de gadolinium, pour les équilibres chimiques suivants : 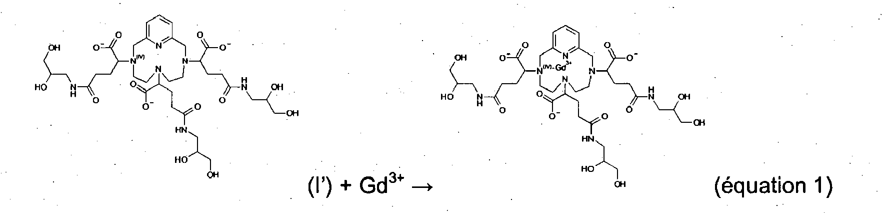 et

DOTA + Gd ↔ [DOTA-Gd] (équation 2)

La constante d'équilibre thermodynamique de l'équation 1 (i.e. pour la complexation du Gd³⁺ par le ligand de formule I') est de 10^{14.9} (i.e. log (Ktherm) = 14.9), tandis que la constante d'équilibre thermodynamique de l'équation 2 (i.e. pour la complexation du Gd³⁺ par le DOTA) est de 10^{25.6} (i.e. log (Ktherm) = 25.6). Ainsi, les formulations selon l'invention vont à l'encontre des enseignements du document US 5,876,695 notamment, puisque le complexe de DOTA avec l'ion de gadolinium est plus stable thermodynamiquement que le complexe actif.

### Modes de réalisation préférés

En particulier, la composition selon l'invention présente une concentration comprise entre 0,001 et 1,5 mol.L⁻¹, préférentiellement comprise entre 0,2 et 0,7 mol.L⁻¹, plus préférentiellement entre 0,3 et 0,6 mol.L⁻¹ en complexe de formule (I) décrit ci-dessus.

Le complexe de formule (I) est dosé par les méthodes connues de l'homme du métier. On peut notamment le doser après une minéralisation et un dosage du métal paramagnétique total présent dans la composition. Dans le cas du dosage du gadolinium total présent dans la solution, le dosage est effectué par spectrométrie d'émission optique (appelée aussi ICP-AES ou ICP Atomic Emission Spectrometry).

La teneur en complexe de formule (I) permet à cette composition d'avoir un pouvoir contrastant optimal tout en ayant une viscosité satisfaisante. En effet, en dessous de 0,01 mol.L⁻¹ de complexe de formule (I) décrit ci-dessus, les performances en tant que produit de contraste sont moins satisfaisantes, et à une concentration supérieure à 1.5 mol.L⁻¹, la viscosité de cette composition devient trop importante pour une manipulation aisée.

Dans un mode de réalisation avantageux, la proportion du complexe de DOTA-calcium est de 0,002 à 5% mole/mole, par exemple de 0.002 à 1 % mole/mole, préférentiellement de 0,01 à 5%, plus préférentiellement de 0,25 à 5% ou de 0,01 à 0,5% mole/mole, encore plus préférentiellement de 0,25 à 0,5% mole/mole, cette proportion étant ramenée à la proportion de complexe de formule (I) dans ladite composition.

Le complexe de DOTA-calcium est dosé également par les méthodes connues de l'homme du métier, par exemple par HPLC (par exemple par HPLC par paire d'ions : à l'aide d'un chromatographe en phase liquide équipé d'un détecteur à barrette d'iode (détection faite par UV à 205 nm) et d'une colonne C18, le solvant utilisé est du méthanol (Prolabo)).

De manière préférentielle, les proportions précisées dans la présente invention et en particulier ci-dessus sont des proportions avant stérilisation de la composition.

De façon avantageuse, le pH de la composition est compris entre 4,5 et 8,5, préférentiellement entre 5 et 6,5. Ces gammes de pH permettent notamment de limiter l'apparition de certaines impuretés et de favoriser la complexation de l'ion de métal paramagnétique M. En particulier, la composition selon l'invention peut être tamponnée, c'est-à-dire qu'elle peut comprendre en outre un tampon choisi parmi les tampons d'usage établi pour la gamme de pH 5 à 6,5 et préférentiellement parmi les tampons lactate, tartrate, malate, maléate, succinate, ascorbate, carbonate, Tris (Tris(hydroxymethyl)aminomethane), HEPES (acide 2-[4-(2-Hydroxyethyl)-1-piperazine]ethanesulfonique), MES (acide 2-morpholino éthanesulphonique) et les mélanges de ceux-ci, et préférentiellement un tampon choisi parmi les tampons lactate, tartrate, carbonate, le MES et les mélanges de ceux-ci.

Du fait notamment du piégeage du métal paramagnétique libre par le DOTA, la composition objet de l'invention peut comprendre en outre un complexe entre du DOTA et un métal, préférentiellement dans une proportion de 0,002 à 0,5% mole/mole, préférentiellement, de 0,01 à 0,5% mole/mole, cette proportion étant ramenée à la proportion de complexe de formule (I) dans ladite composition. Préférentiellement, le complexe entre du DOTA et un métal paramagnétique est un complexe entre du DOTA et un ion de gadolinium (notamment Gd³⁺). La nature du métal chélaté par le DOTA est majoritairement la même que celle du métal paramagnétique chélaté par le ligand du complexe de formule (I). Toutefois, la composition selon l'invention peut en outre comprendre une petite proportion de complexe entre du DOTA et un autre métal que celui chélaté par le ligand du complexe de formule (I). La composition peut ainsi comprendre en outre un complexe entre du DOTA et un ion de tout métal pouvant être extrait des contenants dans lesquels la composition est préparée et/ou stockée, en particulier un ion de fer, de cuivre et/ou de magnésium.

Le complexe entre un ligand chélateur et un lanthanide est préférentiellement complexes de formule:

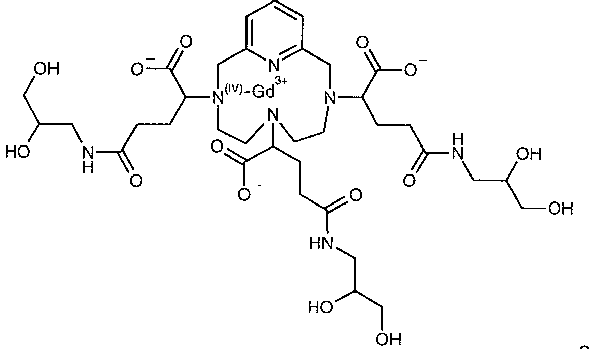

La composition objet de l'invention est préférentiellement stérile.

### Procédé de préparation d'une composition pharmaceutique conforme à l'invention

La présente invention concerne également un procédé de préparation d'une composition selon l'invention. En effet, les métaux paramagnétiques libérés lors de la formulation de ces complexes et/ou lors du stockage de produits de contraste comprenant ces complexes proviennent majoritairement du vieillissement en conservation des chélates, une solution technique a pu être apportée pour permettre un piégeage extrêmement rapide des lanthanides libérés.

Ainsi selon l'invention, le procédé de préparation de la composition pharmaceutique liquide décrite ci-dessus, comprend les étapes successives suivantes :
a) la dissolution du complexe de formule (I) tel que défini précédemment, dans un milieu pharmaceutiquement acceptable,
b) l'addition à la solution obtenue à l'issue de l'étape a) d'une quantité de DOTA libre comprise entre 0,002 et 5% mole/mole par rapport à la quantité de complexe de formule (I) présent dans la composition, et,
c) l'addition à la solution obtenue à l'issue de l'étape b) de 0,002 à 5% mole/mole d'un sel de calcium ou d'oxyde de calcium.

Par « milieu pharmaceutiquement acceptable », on entend, au sens de la présente invention, un milieu compatible avec l'injection intra-veineuse. De manière préférentielle, ce milieu est de l'eau stérile, ou une solution saline stérile, de préférence de l'eau stérile. Ce milieu est préférentiellement tamponné, c'est-à-dire qu'il peut comprendre en outre un tampon choisi parmi les tampons d'usage établi pour la gamme de pH 5 à 6,5 et préférentiellement parmi les tampons lactate, tartrate, malate, maléate, succinate, ascorbate, carbonate, Tris, HEPES, MES et les mélanges de ceux-ci, et préférentiellement un tampon choisi parmi les tampons lactate, tartrate, carbonate, le MES et les mélanges de ceux-ci.

Par « DOTA libre », on entend au sens de la présente invention que le ligand DOTA est présent sous forme non complexée, notamment non complexée à un métal paramagnétique, et n'est pas ajouté sous la forme d'un excipient X[X', DOTA] où X et X' sont un ion métallique ou alcalino-terreux, en particulier choisi indépendamment parmi le calcium, le sodium, le zinc, et le magnésium. En particulier, le DOTA libre n'est pas sous forme de sel, en particulier le DOTA libre n'est pas sous forme de sel de calcium, tel que DOTA-Ca (sel monocalcique), DOTA-Ca₂ (sel dicalcique) ou DOTA-Ca-Na₂ (sel calcium disodique). Un exemple de DOTA libre est sa forme tétraacide (acide 1,4,7,10-tetraazacyclododecane-1,4,7,10-tetraacetique).

Préférentiellement, le sel de calcium est le chlorure de calcium (CaCl₂).

L'étape a) de dissolution du complexe de formule (I) défini précédemment est préférentiellement réalisée en chauffant le milieu pharmaceutiquement acceptable à une température d'au moins 45°C, voire au moins 60°C.

Les étapes b) d'ajout de DOTA libre et/ou c) d'ajout d'un sel de calcium sont avantageusement réalisées, après une diminution de la température de la solution obtenue à l'étape a), à une température de 20 à 40°C. Cette gamme de température est optimale pour limiter les échanges de gadolinium entre le complexe de formule (I) défini précédemment et le DOTA.

Le fait d'ajouter du DOTA non pas sous la forme de sel mais sous forme libre, permet de rendre la complexation du métal paramagnétique libre (Gd) plus efficace. On piège ainsi plus rapidement ce métal paramagnétique que si l'on utilisait, comme décrit dans l'art antérieur, des sels de DOTA.

Dans un mode de réalisation particulièrement avantageux, la quantité (en pourcentage molaire) de sel de calcium ou d'oxyde de calcium ajoutée à la solution à l'étape c) est identique à la quantité de DOTA libre ajouté à l'étape b).

De manière avantageuse, le procédé selon l'invention comprend en outre une étape c') d'ajustement du pH de la solution obtenue à l'étape c), à un pH de 4,5 à 8,5, préférentiellement de 5 à 6,5.

Cette étape c') d'ajustement du pH est préférentiellement réalisée par l'ajout de l'un des tampons cités ci-dessous et/ou par l'ajout d'une solution de soude NaOH 0,1 N ou d'une solution d'acide chlorhydrique HCl 0,1 N.

De manière avantageuse, le procédé selon invention comprend en outre, après l'étape c) ou l'étape c') une étape d) d'ajustement de la concentration dudit complexe de formule (I), après mesure de la masse volumique de la composition, par ajout de milieu pharmaceutiquement acceptable. La concentration finale cible en complexe de formule (I) dans la composition est préférentiellement comprise entre 0,001 à 1,5 mol.L⁻¹, plus préférentiellement comprise entre 0,2 et 0,7 mol.L⁻¹ et encore plus préférentiellement entre 0,3 et 0,6 mol.L⁻¹.

L'étape d'ajustement de la concentration du complexe de formule (I) tel que défini précédemment est préférentiellement une étape d'ajustement du volume par l'ajout de milieu pharmaceutiquement acceptable de façon à ajuster la masse volumique de la composition liquide à une masse volumique préférentiellement comprise entre 0,1 et 1,3 g.cm⁻³, plus préférentiellement de 1,0 à 1,3 g.cm⁻³.

Le procédé selon l'invention peut comprendre en outre une étape de mesure de la quantité de DOTA et/ou de métal paramagnétique en excès à l'issue de l'étape a) et/ou de l'étape b) et/ou de l'étape c) et/ou de l'étape c'), et/ou de l'étape d).

Ces dosages sont réalisés selon des méthodes connues de l'homme du métier. Le dosage du gadolinium est, par exemple, effectué par colorimétrie au Xylénol Orange. Le Xylénol Orange forme avec le gadolinium libre un complexe coloré ayant une absorbance spécifique à la longueur d'onde λ = 567 nm à pH=5,6.

Une étape de stérilisation, avantageusement après l'étape c), c') ou d) du procédé conforme à l'invention, peut aussi avantageusement être ajoutée à ce procédé. Cette stérilisation est réalisée selon les méthodes connues de l'homme du métier. Préférentiellement la composition est stérilisée selon des paramètres sur-destructeurs, c'est-à-dire en termes anglo-saxons selon une approche « overkill ». Cette approche requiert peu d'informations concernant les bio-contaminants de la composition. Par cette approche, on se place dans le cas le plus extrême de bio-contamination et on se place dans les conditions de stérilisation permettant d'obtenir une PNSU (Probability of a Non-Sterile Unit) de 10⁻⁶ pour la composition ainsi stérilisée. Tout procédé de stérilisation qui montre que les indices de létalité Fbio et Fphy (létalité calculée sur la base de paramètres physiques du cycle de stérilisation - C'est l'intégration du taux létal (L) au fil du temps) sont plus grands que 12 minutes convient à la mise en oeuvre de cette approche « overkill ». Un exemple de stérilisation selon une approche « overkill » est une stérilisation par chaleur humide à 121°C pendant 15 minutes (Décision three for the selection of Sterilisation Methods, Annex to Note for Guidance on Development Pharmaceutics (CPMP / QWP/054/98 Corr), EMBA, April 2000). Un autoclave Alphaklave^{®} 23 (HMCE - France) peut être utilisé pour faire cette stérilisation.

Ainsi, dans un mode de réalisation préféré de l'invention, le procédé comprend les étapes successives a), b), c), c'), d), ainsi qu'une étape de stérilisation, lesdites étapes étant telles que définies précédemment.

La concentration de la composition en complexe de formule (I) est typiquement comprise entre 1 mM et 0,6 M. La dose administrée chez le patient est typiquement de l'ordre de 0,01 à 5 mmol/Kg.

### Utilisation des compositions et produits de contraste selon l'invention

L'invention concerne aussi l'utilisation d'une composition selon l'invention pour la préparation d'une composition diagnostique pour imagerie médicale, ou de suivi diagnostique de l'efficacité d'un traitement thérapeutique, et une méthode de diagnostic comprenant l'administration d'une quantité pharmaceutiquement acceptable d'une composition pharmaceutique telle que décrite ci-dessus. L'invention concerne donc un produit de contraste pour l'imagerie médicale comprenant une telle composition pharmaceutique liquide.

L'invention concerne également les compositions ou le produit de contraste décrits précédemment pour leur utilisation pour le diagnostic de maladies, notamment cancéreuses, inflammatoires, neurodégénératives ou vasculaires, en particulier de maladies cardiovasculaires.

L'invention a également trait auxdites compositions ou audit produit de contraste décrits précédemment pour leur utilisation dans une méthode d'imagerie, en particulier une méthode telle que décrite ci-dessous.

Ainsi, l'invention concerne une méthode d'imagerie du corps entier ou d'une partie du corps d'un individu comprenant une étape d'obtention d'une ou plusieurs images du corps entier ou d'une partie du corps d'un individu par une technique d'imagerie médicale, dans laquelle ledit corps entier ou ladite partie du corps de l'individu comprend la composition définie ci-dessus ou le produit de contraste défini ci-dessus (de préférence dans une quantité efficace) et dans laquelle la ou les image(s) sont associée(s) aux particules magnétiques à base d'un composé du fer contenues dans la composition définie ci-dessus ou dans le produit de contraste défini ci-dessus.

Selon un mode de réalisation, la méthode d'imagerie selon l'invention n'inclut pas d'étape d'injection ou d'administration invasive de la composition ou du produit de contraste à l'individu.

Selon un autre mode de réalisation, la méthode d'imagerie selon l'invention comprend une étape préalable d'injection ou d'administration de la composition ou du produit de contraste à l'individu, de préférence une injection par voie intravasculaire.

Dans les méthodes définies ci-dessus, les images sont de préférence obtenues par Imagerie par Résonance Magnétique (ou IRM).

Par « quantité efficace », on entend une quantité de composition selon l'invention ou de produit de contraste comprenant cette composition, qui permet d'obtenir les images par la technique d'imagerie médicale utilisée.

Pour un diagnostic en IRM, l'administration intraveineuse par injection habituellement en solution saline, se fait typiquement à une dose de 1 à 500 µmol Gd/kg. Les doses unitaires pharmaceutiquement acceptables seront fonction de la voie d'administration, ainsi que du patient et notamment de la nature du trouble à étudier.

Pour une injection intraveineuse et une observation par résonance magnétique, la concentration de la solution est comprise typiquement entre 0,001 et 1 mole/litre, et la dose administrée au patient en fonction de son poids sera selon le cas de 0,001 à 0,3 millimole/kilo.

Parmi les indications diagnostiques avantageuses on citera les indications déjà utilisées en clinique, et les indications pour lesquelles les résultats sont améliorés grâce aux formulations. On citera ainsi les indications suivantes et leurs perfectionnements : angiographie, imagerie cérébrale (du système nerveux central notamment), imagerie vasculaire, imagerie de pathologies cardiovasculaires, cancéreuses, neurodégénératives, inflammatoires, toute indication avec imagerie de perfusion, toute indication combinant l'utilisation de plusieurs produits de contraste notamment IRM, scanner aux rayons X, SPECT, PET, PET CT, toute indication avec administrations successives de produits de contraste ou en imagerie multimodale.

Selon des modes de réalisations particuliers, on peut choisir d'administrer les formulations selon l'invention en association ou à la place de formulations de l'art antérieur en fonction du profil diagnostique du patient, et notamment du profil de tolérance du patient aux produits de contraste.

Les compositions de diagnostic de l'invention peuvent comprendre en outre des additifs tels que antioxydants, tampons, régulateurs de l'osmolalité, stabilisants. Des exemples de formulation figurent dans les ouvrages généraux et notamment dans Remington's for Pharmaceutical Science 18e Edition (1990), Mack. Pub. On peut par exemple préparer des solutions aqueuses ou saline stériles comprenant des adjuvants galéniques (lactose, méthylcellulose, mannitol), et/ou des surfactants (lécithines, Tween^{®} ou produits similaires). On pourra aussi utiliser des excipients comme par exemple le mannitol. Une dose pharmaceutiquement acceptable fait référence à une dose appropriée pour une utilisation thérapeutique ou diagnostique.

L'invention sera illustrée à l'aide des exemples non limitatifs qui suivent.

### EXEMPLES DETAILLES

### Exemple 1 : Exemple de procédé de fabrication conforme à l'invention

Le procédé de fabrication d'une composition est réalisé en suivant les étapes suivantes :
a) 485,1 g (soit 0,5 M) de complexe entre un ligand chélateur de formule (I') et un ion de gadolinium (Gd³⁺), qui se présente sous la forme d'une poudre blanche inodore, est dissous dans de l'eau (qs 1 litre) en chauffant la cuve à une température de 50°C et en réalisant une forte agitation de la solution jusqu'à complète dissolution de ce complexe dans l'eau. La solution est ensuite refroidie à environ 30°C. 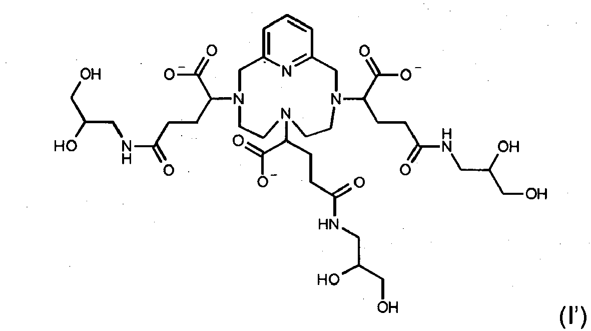
b) 1,011 g (soit 0,5% mole/mole par rapport à la proportion de complexe ajoutée à l'étape a)) de DOTA (Simafex, France) est ajouté à la solution obtenue à l'étape a).
c) 0,368 g (soit 0,5% mole/mole par rapport à la proportion de complexe ajoutée à l'étape a)) de chlorure de calcium (CaCl₂, 2H₂O) (Merck) est ajouté à la solution obtenue à l'étape b).
c') si besoin, le pH de la solution obtenue à l'étape c) est ajusté à un pH de 5 à 6 en diminuant, le cas échéant, le pH par l'ajout d'une solution d'acide chlorhydrique 0,1 N ou en augmentant, le cas échéant, le pH par l'ajout d'une solution de soude 0,1 N.

Dans les proportions de DOTA et de chlorure de calcium telles qu'indiquées ci-dessus, il se forme dans la composition un complexe calcique d'acide 1,4,7,10-tetraazacyclododecane-1,4,7,10-tetraacetique (DOTA) et en particulier majoritairement un complexe d'acide 1,4,7,10-tetraazacyclododecane-1,4,7,10-tetraacetique mono-calcique (DOTA-Ca).

La densité de la composition ainsi obtenue à l'étape c') est ajustée à une valeur de 1,2010 à 1,2219 g.cm⁻³ par l'ajout d'eau. La composition liquide est ensuite filtrée sur une membrane polyethersulfone et mise dans son contenant final, qui est enfin soumis à une stérilisation à 121°C pendant 15 minutes.

### Exemple 2 : Exemple de composition conforme à l'invention et résultats d'études sur celle-ci.

Grâce au procédé de l'exemple 1 est obtenue la formulation suivante :

| **Ingrédients** | **Proportions dans la composition** |
|---|---|
| Complexe de formule (I) dans laquelle M représente un ion de gadolinium (nommé complexe A) | 485,1 g (0,5 M) |
| DOTA | 1,011 g (2,5 mM soit 0,5% mol/mol vs complexe A) |
| CaCl₂, 2H₂O | 0,368 g (2,5 mM soit 0,5% mol/mol vs complexe A) |
| NaOH ou HCl | Qs pH 5,5 ± 0,5 |
| Gadolinium libre* | < 1 ppm m/v |
| Eau ppi (prête pour injection) | Os 1 L |
| * Mesure effectuée par méthode colorimétrique au xylénol orange | |

### Etudes de stabilité en conditions accélérées et sur le long terme

On entend par étude de stabilité en « conditions accélérées », une étude réalisée à 40°C sur 6 mois et par étude de stabilité sur « le long terme », une étude réalisée à 25°C sur 36 mois (Conditions ICH).

Des mesures au cours du temps des deux principales entités en présence dans la composition ont été effectuées.

| | **T0** | | **T 1 mois** | **T 3 mois** | | **T 6 mois** | |
|---|---|---|---|---|---|---|---|
| | Av. s* | Ap. s** | 40°C | 25°C | 40°C | 25°C | 40°C |
| Quantité de gadolinium libre (ppm m/V) | <2 | NA*** | <2 | NA*** | <1 | NA*** | NA*** |
| Quantité de complexe DOTA-Calcium (% mol/mol) | 0,40 | 0,18 | 0,16 | 0,23 | 0,23 | 0.20 | 0,18 |
| *Av.s = Avant stérilisation | | | | | | | |
| **Ap.s = Après stérilisation | | | | | | | |
| ***NA = Non analysé | | | | | | | |

Il n'est ni détecté, ni quantifié du gadolinium libre dans la composition. La quantité de complexe DOTA-Calcium diminue de manière significative du fait de la stérilisation mais reste stable après six mois en conditions accélérées et sur le long terme. Environ la moitié du complexe DOTA-Ca qui s'est formé dans la composition après ajout du DOTA et du sel de calcium reste disponible dans la composition pour assurer sa fonction de piégeage du gadolinium libre.

### Etudes de stabilité en fonction de la proportion en DOTA-Ca

Plusieurs autres formulations ont été réalisées avec une proportion croissante de DOTA-Ca (en particulier de complexe monocalcique) par rapport au complexe A. La concentration en lanthanide libre et la concentration en DOTA-Ca, ont été mesurées après l'étape de stérilisation et après 3 mois de stockage à 25 °C et 40 °C.

| **% mol/mol de DOTA-Ca vs complexe A** | | | **Quantité de gadolinium libre (ppm m/v)** | **Quantité de complexe DOTA-Ca (%mol/mol)** |
|---|---|---|---|---|
| 0,01 | T0 | Ap.s* | <0,50 | ND" |
| | T3 mois | 25 °C | <0,50 | ND** |
| | | 40 °C | <0,50 | ND" |
| 0,25 | T0 | Ap.s* | <0,50 | 0,14 |
| | T3 mois | 25 °C | <0,50 | 0,16 |
| | | 40 °C | <0,50 | 0,16 |
| 0,5 | T0 | Ap.s* | <0,50 | 0,34 |
| | T3 mois | 25 °C | <0,50 | 0,33 |
| | | 40 °C | <0.50 | 0,30 |
| 0,75 | T0 | Ap.s* | <0,50 | 0,53 |
| | T3 mois | 25 °C | <0,50 | 0,57 |
| | | 40 °C | <0,50 | 0,51 |
| 5 | T0 | Ap.s* | <0,50 | 4,60 |
| | T3 mois | 25 °C | <0,50 | 4,80 |
| | | 40 °C | <0,50 | 4,80 |
| Ap.s* : Après stérilisation | | | | |
| ND** : Non détecté | | | | |

Pour une proportion de DOTA-Ca comprise entre 0,01 et 5% mol/mol par rapport au complexe A, la concentration en Gd³⁺ libre est inférieure à 0.5 ppm (m/v).

La diminution de la teneur en DOTA-Ca avec le temps traduit une consommation de l'excipient de formulation. Néanmoins, pour une proportion initiale en DOTA-Ca supérieure ou égale à 0,25% mol/mol par rapport au complexe A, la quantité de DOTA-Ca disponible après 3 mois de stockage à 40 °C reste, au minimum, supérieure à plus de la moitié de la quantité initialement introduite. Cet excès d'excipient de formulation apporte une garantie supplémentaire en terme de captation du gadolinium libéré par le complexe A au cours du stockage du produit.

### Exemple 3 : Comparaison du procédé de fabrication selon l'invention et de procédés de l'art antérieur

### Résultats de dosage de gadolinium dans les compositions de PCTA - Gd après ajout d'une solution de DOTA-Calcium ou après ajout de DOTA puis de CaCl₂ (conformément à l'invention)

Une solution mère de complexe entre le ligand chélateur de formule (I') et un ion de gadolinium enrichie en gadolinium libre est utilisée.

Dans cette solution est ajoutée soit du DOTA sous forme de poudre puis du CaCl₂ (procédé conforme à l'invention), soit une solution de DOTA-calcium ajustée à pH 6,0 (en extrapolant à partir des procédés décrits dans l'art antérieur).

| | **Quantité de Gd³⁺** | |
|---|---|---|
| | **Ajout de DOTA sous forme de poudre puis du CaCl₂** | **Ajout d'une solution de DOTA-calcium ajustée à pH 6,0** |
| Solution mère de complexe entre le ligand chélateur de formule (I') et un ion de gadolinium enrichie en gadolinium libre | 73 ppm m/v | 73 ppm m/v |
| 1h après ajout du DOTA sous forme de poudre et ajustement de pH | < 1 ppm m/v | - |
| 5 minutes après l'ajout de DOTA calcium | - | 3 ppm m/v |
| 1h après l'ajout de DOTA calcium | - | 3 ppm m/v |
| 1h30 après l'ajout de DOTA calcium | - | 3 ppm m/v |
| 2h15 après l'ajout de DOTA calcium | - | 3 ppm m/v |
| Avant stérilisation | < 1 ppm m/v | 4 ppm m/v |
| Après stérilisation | < 1 ppm m/v | < 3 ppm m/v |

L'ajout successif de DOTA sous forme de poudre puis du chlorure de calcium CaCl₂ permet de complexer efficacement tout le Gd³⁺ présent dans la solution de complexe de ligand chélateur de formule (I') et du gadolinium, enrichie en gadolinium libre. En effet, la quantité importante de Gd³⁺ dans la solution mère n'est plus détectée une heure après l'ajout du DOTA.

A l'inverse, lorsqu'un complexe DOTA-Calcium préalablement formé est ajouté sous forme de solution, des ions de gadolinium Gd³⁺ persistent même après avoir maintenu l'agitation pendant 2h15.

Le mode d'ajout du DOTA-calcium a donc une influence sur la quantité de Gd³⁺ en solution.

### Résultats de dosage du DOTA-calcium dans les compositions de PCTA - Gd après ajout d'une solution de DOTA-Calcium ou après ajout de DOTA puis de CaCl₂ (conformément à l'invention)

| | **Quantité de DOTA-Ca (en % mol/mol)** | |
|---|---|---|
| | **Ajout de DOTA sous forme de poudre puis du CaCl₂** | **Ajout d'une solution de DOTA-calcium ajustée à pH 6,0** |
| Après ajout du DOTA sous forme de poudre et ajustement de pH | 0.27 | - |
| 1h après l'ajout de DOTA calcium | - | 0,47 |
| 1 h30 après l'ajout de DOTA calcium | - | 0,40 |
| Avant stérilisation | 0,25 | 0,37 |
| Après stérilisation | 0,25 | 0,40 |

La consommation du DOTA-Ca est plus importante lorsque le DOTA est ajouté sous forme de poudre que lorsqu'il est ajouté directement sous forme de complexe DOTA-Ca. Lorsque le DOTA est ajouté sous forme de poudre, il est capable de complexer directement le Gd³⁺ en solution. Puis le CaCl₂ se complexe avec le DOTA libre restant ce qui explique la quantité plus faible de DOTA-Ca. Quand l'ajout est effectué directement sous forme de complexe DOTA-Ca, il faut qu'il y ait échange du gadolinium et du Ca (Complexe-Gd + DOTA-Ca ⇄ Complexe-Ca + DOTA-Gd). La réaction est moins rapide et elle n'est pas totale puisque des traces de Gd³⁺ sont présentes plus de 2h après l'ajout de DOTA-Calcium. La consommation de DOTA-Ca est peu importante lors de la stérilisation pour les deux procédés de fabrication.

## Revendications

1. Composition pharmaceutique liquide comprenant un complexe de formule (I) : 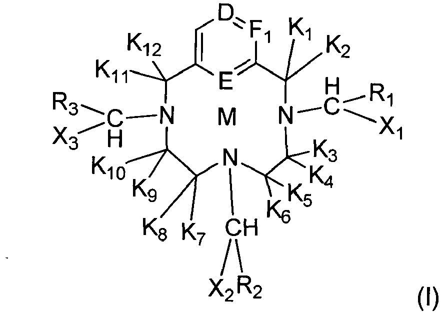
dans laquelle R₁, R₂ et R₃ représentent -COOH,
X₁, X₂ et X₃ représentent indépendamment l'un de l'autre L-Y dans laquelle L représente un groupe (CH₂)ₙ avec n = 1 à 3, Y représente -CO-NR₇Rₛ dans laquelle R₇ représente H et R₈ représente un groupe -CH₂-CH₂OH, -CHOH-CH₂OH, -CH-(CH₂OH)₂, - CH₂-(CHOH)ₚ-CH₂OH avec p = 1 à 4 ou -C-(CH₂OH)₃ ;
D représente CH;
E représente N;
F₁ représente CH;
K₁ à K₁₂ représentent chacun ; et
M représente Gd³⁺ ;
ou un énantiomère, ou un diastéréoisomère (préférentiellement choisi parmi les diastéréoisomères RRS, RSR, RSS) de ceux-ci ou leurs mélanges,
ladite composition comprenant en outre un complexe calcique d'acide 1,4,7,10-tetraazacyclododecane-1,4,7,10-tetraacetique et ayant une concentration en métal paramagnétique libre inférieure à 1 ppm (m/v), préférentiellement inférieure à 0,5 ppm (m/v).

2. Composition selon la revendication précédente, **caractérisée en ce qu'**elle présente une concentration comprise entre 0,001 et 1,5 mol.L⁻¹ dudit complexe de formule (I).

3. Composition selon l'une quelconque des revendications précédentes, **caractérisée en ce que** la proportion de DOTA-Ca est de 0,002 à 5% mole/mole, cette proportion étant ramenée à la proportion de complexe de formule (I) dans ladite composition.

4. Composition selon l'une quelconque des revendications précédentes, **caractérisée en ce que** son pH est compris entre 4,5 et 8,5, préférentiellement entre 5 et 6,5.

5. Composition selon l'une quelconque des revendications précédentes, **caractérisée en ce qu'**elle comprend en outre un tampon choisi parmi les tampons lactate, tartrate, malate, maléate, succinate, ascorbate, carbonate, Tris, HEPES, MES et les mélanges de ceux-ci.

6. Composition selon l'une quelconque des revendications précédentes, **caractérisée en ce que** le complexe de formule (I) est

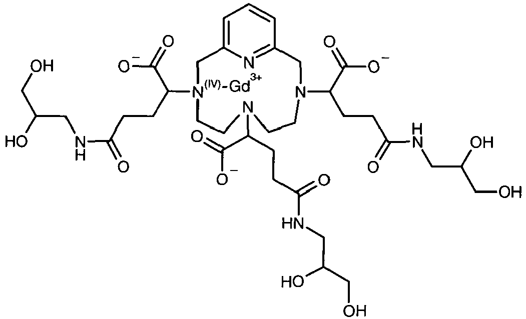

7. Composition selon l'une quelconque des revendications précédentes, **caractérisée en ce qu'**elle est stérile.

8. Produit de contraste pour l'imagerie médicale comprenant la composition pharmaceutique liquide selon l'une quelconque des revendications 1 à 7.

9. Méthode d'imagerie du corps entier ou d'une partie du corps d'un individu comprenant une étape d'obtention d'une ou plusieurs images du corps entier ou d'une partie du corps d'un individu par une technique d'imagerie médicale, dans laquelle ledit corps entier ou ladite partie du corps de l'individu comprend la composition selon l'une quelconque des revendications 1 à 7 οu le produit de contraste selon la revendication 8 et dans laquelle la ou les image(s) sont associée(s) au complexe entre un ligand chélateur de formule (I) telle que défini dans la revendication 1 et un métal paramagnétique contenu dans la composition selon l'une quelconque des revendications 1 à 7 ou dans le produit de contraste selon la revendication 8.

10. Procédé de préparation d'une composition pharmaceutique liquide telle que définie à la revendication 1, ledit procédé comprenant les étapes successives suivantes :
a) la dissolution du complexe tel que défini dans la revendication 1, dans un milieu pharmaceutiquement acceptable,
b) l'addition à la solution obtenue à l'issue de l'étape a) d'une quantité de DOTA libre comprise entre 0,002 et 5% mole/mole par rapport à la quantité de complexe de formule (I) présent dans la composition et,
c) l'addition à la solution obtenue à l'issue de l'étape b) de 0,002 à 5% mole/mole d'un sel de calcium ou d'oxyde de calcium.

11. Procédé selon la revendication 10, **caractérisé en ce qu'**il comprend en outre une étape c') d'ajustement du pH de la solution obtenue à l'étape b) à un pH de 4,5 à 8,5.

12. Procédé selon l'une quelconque des revendications 10 à 11, **caractérisé en ce qu'**il comprend en outre une étape de stérilisation.

13. Composition pharmaceutique liquide obtenue selon le procédé selon l'une quelconque des revendications 10 à 12.

## Patentansprüche

1. Flüssige pharmazeutische Zusammensetzung, umfassend einen Komplex der Formel (I): 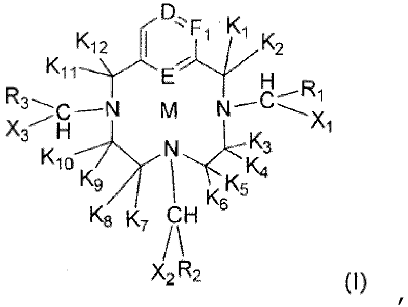
worin R₁, R₂ und R₃ für -COOH stehen,
X₁, X₂ und X₃ unabhängig voneinander L-Y bedeuten, wobei L für eine (CH₂)ₙ-Gruppe mit n = 1 bis 3 steht und Y für -CO-NR₇R₈ steht, wobei R₇ für H und R₈ für eine -CH₂-CH₂OH-, -CHOH-CH₂OH-, -CH-(CH₂OH)₂-, -CH₂-(CHOH)ₚ-CH₂OH-Gruppe mit p = 1 bis 4 oder -C-(CH₂OH)₃-Gruppe steht;
D für CH steht;
E für N steht;
F₁ für CH steht;
K₁ bis K₁₂ jeweils für H stehen; und
M Gd³⁺ darstellt;
oder ein Enantiomer oder ein Diastereoisomer (das vorzugsweise aus RRS-, RSR-, RSS-Diastereomeren ausgewählt ist) von diesem oder ihre Gemische,
wobei die Zusammensetzung außerdem einen Calciumkomplex von 1,4,7,10-Tetraazacyclododecan-1,4,7,10-tetraessigsäure enthält und eine Konzentration an freiem paramagnetischem Metall unter 1 ppm (w/v), vorzugsweise unter 0,5 ppm (w/v), aufweist.

2. Zusammensetzung nach dem vorhergehenden Anspruch, **dadurch gekennzeichnet, dass** sie eine Konzentration zwischen 0,001 und 1,5 mol.l⁻¹ des Komplexes der Formel (I) aufweist.

3. Zusammensetzung nach einem der vorhergehenden Ansprüche, **dadurch gekennzeichnet, dass** der Anteil an DOTA-Ca 0,002 bis 5% Mol/Mol beträgt, wobei dieses Verhältnis auf den Anteil an Komplex der Formel (I) in der Zusammensetzung bezogen ist.

4. Zusammensetzung nach einem der vorhergehenden Ansprüche, **dadurch gekennzeichnet, dass** ihr pH-Wert zwischen 4,5 und 8,5, vorzugsweise zwischen 5 und 6,5, beträgt.

5. Zusammensetzung nach einem der vorhergehenden Ansprüche, **dadurch gekennzeichnet, dass** sie außerdem einen aus Lactat-, Tartrat-, Malat-, Maleat-, Succinat-, Ascorbat-, Carbonat-, Tris-, HEPES- und MES-Puffern und deren Gemischen ausgewählten Puffer umfasst.

6. Zusammensetzung nach einem der vorhergehenden Ansprüche, **dadurch gekennzeichnet, dass** der Komplex der Formel (I): 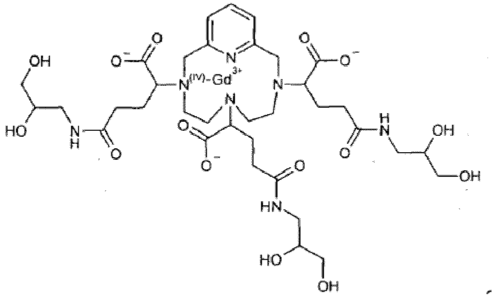 ist.

7. Zusammensetzung nach einem der vorhergehenden Ansprüche, **dadurch gekennzeichnet, dass** sie steril ist.

8. Kontrastmittel für die medizinische Bildgebung, dass die flüssige pharmazeutische Zusammensetzung nach einem der Ansprüche 1 bis 7 umfasst.

9. Verfahren zur Bildgebung des gesamten Körpers oder eines Teils des Körpers eines Individuums, umfassend einen Schritt, in dem ein oder mehrere Bilder des gesamten Körpers oder eines Teils des Körpers eines Individuums durch eine medizinische Bildgebungstechnik erhalten werden, wobei der gesamte Körper oder der Teil des Körpers des Individuums die Zusammensetzung nach einem der Ansprüche 1 bis 7 oder das Kontrastmittel nach Anspruch 8 umfasst und wobei das oder die Bild(er) mit dem Komplex zwischen einem chelatbildenden Liganden der Formel (I) nach Anspruch 1 und einem in der Zusammensetzung nach einem der Ansprüche 1 bis 7 oder dem Kontrastmittel nach Anspruch 8 enthaltenen paramagnetischen Metall in Beziehung gesetzt wird/werden.

10. Verfahren zur Herstellung einer flüssigen pharmazeutischen Zusammensetzung nach Anspruch 1, wobei das Verfahren die folgenden aufeinanderfolgenden Schritte umfasst:
a) Lösen des Komplexes nach Anspruch 1 in einem pharmazeutisch annehmbaren Medium,
b) Zugabe zu der Lösung, die am Ende von Schritt a) erhalten wird, einer Menge an freiem DOTA zwischen 0,002% und 5% Mol/Mol, bezogen auf die in der Zusammensetzung vorhandene Menge an Komplex der Formel (I), und
c) Zugabe zu der Lösung, die am Ende von Schritt b) erhalten wird, von 0,002% bis 5% Mol/Mol eines Calciumsalzes oder Calciumoxids.

11. Verfahren nach Anspruch 10, **dadurch gekennzeichnet, dass** es außerdem einen Schritt c') umfasst, in dem der pH der in Schritt b) erhaltenen Lösung auf einen pH von 4,5 bis 8,5 eingestellt wird.

12. Verfahren nach einem der Ansprüche 10 bis 11, **dadurch gekennzeichnet, dass** es außerdem einen Sterilisierungsschritt umfasst.

13. Flüssige pharmazeutische Zusammensetzung, die gemäß dem Verfahren nach einem der Ansprüche 10 bis 12 erhalten wird.

## Claims

1. Liquid pharmaceutical composition comprising a complex of formula (I): 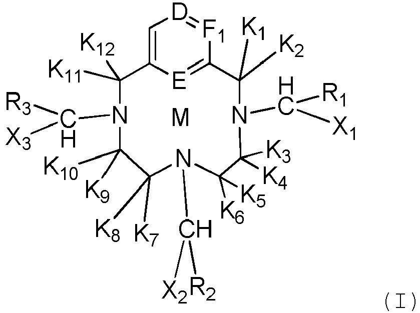
in which R₁, R₂ and R₃ represent -COOH,
X₁, X₂ and X₃ represent, independently of one another, L-Y in which L represents a (CH₂)ₙ group with n = 1 to 3, and Y represents -CO-NR₇R₈, in which R₇ represents H and R₈ represents a -CH₂-CH₂OH, -CHOH-CH₂OH, -CH-(CH₂OH)₂, -CH₂-(CHOH)ₚ-CH₂OH group with p = 1 to 4 or -C-(CH₂OH)₃;
D represents CH;
E represents N;
F₁ represents CH;
K₁ to K₁₂ each represent H; and
M represents Gd³⁺;
or an enantiomer or a diastereoisomer (preferentially chosen from the RRS, RSR and RSS diastereoisomers) thereof or mixtures thereof,
said composition also comprising a calcium complex of 1,4,7,10-tetraazacyclododecane-1,4,7,10-tetraacetic acid, and having a concentration of free paramagnetic metal of less than 1 ppm (m/v), preferentially less than 0.5 ppm (m/v).

2. Composition according to the preceding claim, **characterized in that** it has a concentration of said complex of formula (I) of between 0.001 and 1.5 mol.l⁻¹.

3. Composition according to either one of the preceding claims, **characterized in that** the proportion of DOTA-Ca is from 0.002% to 5% mol/mol, this proportion being related back to the proportion of complex of formula (I) in said composition.

4. Composition according to any one of the preceding claims, **characterized in that** its pH is between 4.5 and 8.5, preferentially between 5 and 6.5.

5. Composition according to any one of the preceding claims, **characterized in that** it also comprises a buffer chosen from lactate, tartrate, malate, maleate, succinate, ascorbate, carbonate, Tris, HEPES and MES buffers and mixtures thereof.

6. Composition according to any one of the preceding claims, **characterized in that** the complex of formula (I) is:

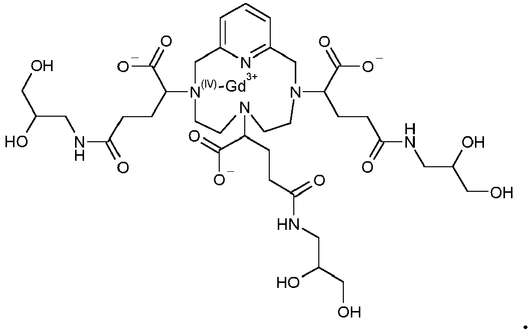

7. Composition according to any one of the preceding claims, **characterized in that** it is sterile.

8. Contrast product for medical imaging, comprising the liquid pharmaceutical composition according to any one of Claims 1 to 7.

9. Method for imaging the whole body or a part of the body of an individual, comprising a step of obtaining one or more images of the whole body or of a part of the body of an individual by means of a medical imaging technique, in which said whole body or said part of the body of the individual comprises the composition according to any one of Claims 1 to 7 or the contrast product according to Claim 8 and in which the image(s) is (are) associated with the complex between a chelating ligand of formula (I) as defined in Claim 1 and a paramagnetic metal contained in the composition according to any one of Claims 1 to 7 or in the contrast product according to Claim 8.

10. Process for preparing a liquid pharmaceutical composition as defined in Claim 1, said process comprising the following successive steps:
a) dissolution of the complex as defined in Claim 1, in a pharmaceutically acceptable medium,
b) addition, to the solution obtained at the end of step a), of an amount of free DOTA of between 0.002% and 5% mol/mol relative to the amount of complex of formula (I) present in the composition, and
c) addition, to the solution obtained at the end of step b), of 0.002% to 5% mol/mol of a calcium salt or of calcium oxide.

11. Process according to Claim 10, **characterized in that** it also comprises a step c') of adjustment of the pH of the solution obtained in step b) to a pH of 4.5 to 8.5.

12. Process according to either one of Claims 10 and 11, **characterized in that** it also comprises a sterilization step.

13. Liquid pharmaceutical composition obtained according to the process according to any one of Claims 10 to 12.
